(19) Europäisches Patentamt
European Patent Office
Office européen des brevets

(11) Veröffentlichungsnummer: **0 393 499**
**A1**

(12) # EUROPÄISCHE PATENTANMELDUNG

(21) Anmeldenummer: **90106989.8**

(51) Int. Cl.5: **C07D 473/18, C07D 239/50**

(22) Anmeldetag: **11.04.90**

(30) Priorität: **20.04.89 US 340648**

(43) Veröffentlichungstag der Anmeldung:
**24.10.90 Patentblatt 90/43**

(84) Benannte Vertragsstaaten:
**AT BE CH DE DK FR GB IT LI NL**

(71) Anmelder: **F. HOFFMANN-LA ROCHE AG**
**Postfach 3255**
**CH-4002 Basel(CH)**

(72) Erfinder: **Schwartz, Alan**
**10 Watchung Avenue**
**Upper Montclair, N.J. 07043(US)**

(74) Vertreter: **Grossner, Lutz, Dr. et al**
**Grenzacher Strasse 124 Postfach 3255**
**CH-4002 Basel(CH)**

(54) **Verfahren zur Herstellung von Carbovir und neue Zwischenprodukte dafür.**

(57) Die Erfindung betrifft ein neues Verfahren zur Herstellung des anti-Aids-Mittels Carbovir und dabei verwendete neue Zwischenprodukte.

EP 0 393 499 A1

## Verfahren zur Herstellung von Carbovir und neue Zwischenprodukte dafür

Kürzlich haben Vince et al. [Biochem. Biophys. Res. Commun. 156, 1046 (1988)] ein carbocyclisches Nukleosidanalog von Guanosin beschrieben, das ein wirksamer und selektiver Hemmer der HIV-Replikation in vitro ist. Dieses Analog, carbocyclisches 2′,3′-Didehydro-2′,3′-dideoxyguanosin, ist gemeinhin als Carbovir bekannt.

Die vorliegende Erfindung betrifft neue Zwischenprodukte für die Synthese von Carbovir, Verfahren zur Herstellung dieser Zwischenprodukte und Verfahren für die Synthese von Carbovir, bei der diese Zwischenprodukte verwendet werden. Mittels der erfindungsgemässen Zwischenprodukte und Verfahren kann Carbovir einfacher und in viel höherer Ausbeute als bisher möglich hergestellt werden.

Die Erfindung betrifft insbesondere ein Verfahren zur Herstellung von Carbovir, dadurch gekennzeichnet, dass man

a) eine Verbindung der Formel

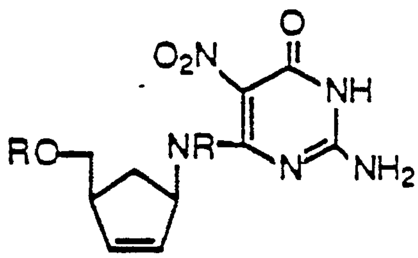

III

worin R Wasserstoff oder eine mit Säure spaltbare Schutzgruppe darstellt,
mit wässrigem Natriumdithionit in Gegenwart eines Alkanols und eines polaren aprotischen Lösungsmittels unter Erhitzen zum Rückfluss zu einer Verbindung der Formel

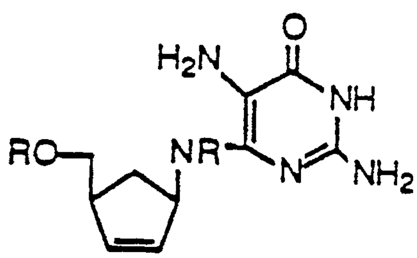

IV

worin R die obige Bedeutung hat,
reduziert und

b) die Verbindung der Formel IV durch

(i) Behandlung mit einem Formylierungsmittel in einem polaren aprotischen Lösungsmittel in Gegenwart eines nicht-wässrigen sauren Katalysators unter Erhitzen zum Rückfluss und

(ii) Dehydratisieren der formylierten Verbindung mit einer Mineralsäure

cyclisiert.

Die Erfindung betrifft weiterhin ein Verfahren zur Herstellung von Verbindungen der Formel III, dadurch gekenn zeichnet, dass man eine Verbindung der Formel

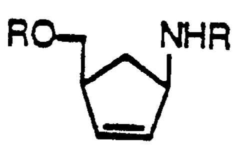

I

worin R die obige Bedeutung hat,
mit einer Verbindung der Formel

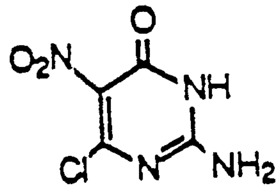

II

in einem polaren aprotischen Lösungsmittel in Gegenwart eines tertiären Alkylamins umsetzt.

Die Zwischenprodukte der Formel III und IV sind neue Verbindungen und ebenfalls Gegenstand der Erfindung.

Die neuen erfindungsgemässen Verfahren und Verbindungen gründen sich auf die Anwendung von zwei Ausgangsstoffen, die Verbindungen der Formel I und II. Die Verbindung der Formel I, in der R Wasserstoff ist, kann dadurch hergestellt werden, dass man Methyl cis-4-acetamidocyclopent-2-encarboxylat in Gegenwart von $Ba(OH)_2 \cdot 8H_2O$ wie unten beschrieben zum Rückfluss erhitzt. Die Verbindung der Formel II kann aus handelsüblichem 2-Amino-4,6-dichlorpyrimidin wie von Constant et al. [J. Heterocyclic Chem. 22, 1035 (1985)] beschrieben hergestellt werden.

Die Umsetzung der Verbindungen der Formel I und II unter Bildung der Verbindung der Formel III wird unter wasserfreien Bedingungen in einem polaren aprotischen Lösungsmittel in Gegenwart eines tertiären Alkylamins durchgeführt. Geeignete polare aprotische Lösungsmittel sind beispielsweise Dimethylacetamid, Dimethylsulfoxid und Dimethylformamid. Beispiele tertiärer Alkylamine, die angewandt werden können, sind Trimethylamin, Triäthylamin, Tributylamin und Diisopropyläthylamin. Die Reaktion kann bei einer Temperatur von etwa 0 bis etwa 80 °C durchgeführt werden, zweckmässig wird sie bei Raumtemperatur ausgeführt.

Obschon aus ökonomischen Gründen der Einsatz äquimolarer Verhältnisse der Verbindungen der Formel I und II und dem tertiären Amin naheliegt, wird die Reaktion vorzugsweise mit etwa 1,5 Aequivalenten der Verbindung der Formel I durchgeführt, um eine vollständige Umsetzung der Verbindung der Formel II zu gewährleisten und die Reinigung des Verfahrensproduktes zu erleichtern.

Die Reduktion der Nitrogruppe der Verbindung der Formel III unter Bildung der Formel IV wird in einem Gemisch von wässrigem Natriumdithionit, einem niederen Alkanol und einem polaren aprotischen Lösungsmittel in etwa gleichen Volumenanteilen durchgeführt. Niedere Alkanole, die hier verwendet werden können, sind beispielsweise solche, die etwa fünf C-Atome oder weniger haben, wie Methanol, Aethanol, Propanol, Isopropanol oder Butanol. Geeignete polare aprotische Lösungsmittel sind die oben beschriebenen. Die Reaktion wird zweckmässig unter Rückfluss ausgeführt.

Die Reduktion kann bei einer Temperatur von etwa 25 bis etwa 100 °C durchgeführt werden, wird aber zweckmässig bei Rückflusstemperatur durchgeführt. Vorzugsweise wird sie unter Verwendung eines Verhältnisss von etwa 2 bis etwa 5 Aequivalenten Dithionit, bezogen auf die Verbindung der Formel III, in wässrigem Dimethylformamid bei Rückflusstemperatur durchgeführt.

Die so hergestellte Diaminoverbindung der Formel IV wurde mittels ihrer Spektraleigenschaften identifiziert, war jedoch, was für solche Verbindungen charakteristisch ist, instabil. Diese Verbindung wird somit vorzugsweise ohne sie zu isolieren hergestellt und eingesetzt. Die Cyclisierung der Verbindung der Formel IV wird in zwei Stufen ausgeführt.

Zunächst wird die Aminogruppe in der 5-Stellung des heterocyclischen Rings mit einem Formylierungsmittel in einem polaren aprotischen Lösungsmittel in Gegenwart eines nicht-wässrigen sauren Katalysators behandelt. Geeignete polare aprotische Lösungsmittel sind die oben beschriebenen. Geeignete Formylierungsmittel sind Triäthylorthoformat, Trimethylorthoformat und Dialkylacetale wie Dimethyl- oder Diäthylacetal. Ein nicht-wässriger sauren Katalysators wie p-Toluolsulfonsäure wird angewandt und die Reaktion wird zweckmässig bei Rückflusstemperaturen ausgeführt. Die Formylierung wird geeigneter Weise unter Bedingungen ausgeführt, bei denen das Formylierungsmittel, z.B. Triäthylorthoformat, als Lösungsmittel eingesetzt wird, das rohe Reduktionsprodukt der Formel IV wird in einer minimalen Menge Dimethylformamid gelöst und das Gemisch wird mit dem sauren Katalysator über Nacht zum Rückfluss erhitzt.

In der zweiten Stufe wird das formylierte Zwischenprodukt mit einer Mineralsäure, wie Chlorwasserstoffsäure, Schwefelsäure oder Phosphorsäure dehydratisiert, wobei der neue Ring gebildet wird.

Die vorstehenden Reaktionen können unter Verwendung der Verbindung der Formel I, III und IV durchgeführt werden, in denen R eine mit Säure spaltbare Schutzgruppe wie Trialkylsilyl darstellt. Beispiele solcher Gruppen sind t-Butyldimethylsilyl, t-Butyldiphenylsilyl, Triphenylsilyl, Triäthylsilyl und Diphenylmethylsilylgruppen. Solche Verbindungen können leicht durch Umsetzung einer Verbindung der Formel I, in der R Wasserstoff ist, mit dem entsprechenden Trialkylsilylchlorid in Gegenwart eines tertiären Amins in äquimolaren Mengen in einem nicht-protischen Lösungsmittel, wie Dichlormethan, Toluol, Aether oder Tetrahydrofuran hergestellt werden. Die Reaktion kann bei Temperaturen von etwa 0 °C bis zur Rückflusstemperatur ausgeführt werden, obschon Raumtemperatur zweckmässig ist. Sie können auch durch Umsetzung einer Verbindung der Formel I, in der R Wasserstoff ist, mit Hexamethyldisilazan unter Rückflusserhitzen hergestellt werden.

Bei der Herstellung von Carbovir wird die Schutzgruppe während der Behandlung mit Mineralsäure abgespaltet.

Die Erfindung wird durch die nachstehenden Beispiele weiter erläutert.

## Beispiel 1

Methyl cis-4-acetamidocyclopent-2-encarboxylat

Zu 2,4 l frisch destilliertem Cyclopentadien werden bei 0 °C 340,9 g Tosylcyanid auf ein Mal zugegeben und die Suspension wird rasch ohne äussere Kühlung 2 Stunden gerührt. Die erhaltene hellgelbe durchsichtige Lösung wurde bei 35 °C zu einem dicken weissen granulösen Rückstand eingedampft.

Der Rückstand wurde auf unter 0 °C 15 Minuten lang unter Argon abgekühlt, mit 500 ml kalter (etwa 17 °C) Eisessig versetzt und das Gemisch 10 Minuten bei Raumtemperatur gerührt. Das hellgelbe Gemisch wurde auf etwa 5 °C gekühlt, mit 600 ml kalter 4N HCl versetzt und das Gemisch 3 Stunden bei 5 °C und dann über Nacht bei Raumtemperatur gerührt. Das Gemisch wurde nach und nach dunkelbraun und Feststoffe fielen aus.

Die Lösung wurde durch Celit filtriert und mit dreimal 250 ml Wasser gewaschen. Die vereinigten wässrigen Phasen wurden mit dreimal 500 ml Aether extrahiert und die vereinigten Aetherextrakte mit 250 ml Wasser rückextrahiert. Die vereinigten wässrigen Phasen wurde zu einem gelben Rückstand konzentriert, der mit dreimal 600 ml Toluol azeotrop abgedampft wurde, wobei ein trockener amorpher Rückstand erhalten wurde.

Der Rückstand wurde in 1,5 l absolutem Methanol gelöst. In die Lösung wurde 5 Minuten HCl-Gas bis zur Sättigung eingeleitet und das Gemisch über Nacht zum Rückfluss erhitzt. Die dann dunkle Lösung wurde zu einem dunklen Rückstand konzentriert, der mit 1,2 l Pyridin versetzt wurde. Die dunkle Lösung wurde in einem Eisbad auf 5 °C gekühlt und sodann mit 750 ml Acetanhydrid versetzt. Das Kühlbad wurde entfernt und die Lösung über Nacht gerührt.

Die dunkle Lösung wurde zu einem dunklen braunen Sirup eingedampft. Der Sirup wurde in 1,2 l $CH_2Cl_2$ gelöst und zuerst mit zweimal 1 l Wasser und danach mit dreimal 500 ml 1,5N $H_2SO_4$ extrahiert, worauf die organische Phase über Kaliumcarbonat getrocknet wurde.

Nach Eindampfen des Lösungsmittels wurden 364,4 g kristallines Rohprodukt erhalten. Dieses Rohprodukt wurde an 300 g Silicagel adsorbiert und auf ein Bett von 300 g Silicagel gegeben. Elution mit Aethylacetat, Sammeln der homogenen Fraktionen und Eindampfen des Lösungsmittels lieferte 230 g Produkt vom Schmelzpunkt 57-59 °C. Die Gesamtausbeute aus Tosylcyanid betrug 67%. IR, NMR und MS entsprachen der erwarteten Struktur.

## Beispiel 2

(±)-(1α,4α)-4-[(2-Amino-1,6-dihydro-5-nitro-6-oxo-4-pyrimidinyl)amino]-2-cyclopentencarbinol

Ein Gemisch von 14,65 g Methyl cis-4-acetamidocyclopent-2-encarboxylat in 1,2 l Wasser und 120,0 g $Ba(OH)_2 \cdot 8H_2O$ wurde über Nacht zum Rückfluss erhitzt. Am nächsten Tag wurde das Gemisch auf Raumtemperatur gekühlt und mit überschüssigem Trockeneis versetzt, um es zu neutralisieren. Das ausgefällte $BaCO_3$ wurde abfiltriert und die wässrige Lösung zu einem hellbraunen Oel konzentriert. Zusatz von 100 ml absolutem Aethanol bewirkte wiederum eine Ausfällung von Feststoffen, die abfiltriert wurden,

und das Filtrat wurde wie vorher zu einem hellbraunen Oel konzentriert.

Das Oel wurde in 100 ml wasserfreiem DMF (Dimethylformamid, getrocknet durch Aufbewahren über 3A Molekularsieb während mehr als 30 Tagen) gelöst und dann mit 35 ml Triäthylamin versetzt. Danach wurden 11,6 g 2-Amino-6-chlor-5-nitro-4(3H)-pyrimidinon zugesetzt und das homogene Gemisch wurde über Nacht bei Raumtemperatur gerührt. Das Gemisch wurde zu einem dunklen Oel konzentriert, mit 110 ml Wasser versetzt, 15 Minuten stehen gelassen und dann filtriert, wobei das Rohprodukt als brauner Feststoff gesammelt wurde. Der Feststoff wurde in 300 ml Aceton suspendiert, zum Rückfluss erwärmt, auf Raumtemperatur gekühlt, filtriert und mit 25 ml Aceton gewaschen.

Nach Trocknen bei 20 °C/26,6 mbar erhielt man 12,65 g Produkt vom Schmelzpunkt 205-207 °C, das gemäss TLC (4:1 $CHCl_3$:MeOH) homogen war. Die Mutterlaugen wurden zur Trockene eingedampft und in 15 ml Aceton suspendiert. Nach Waschen und Trocknen der Feststoffe wurden weitere 0,66 g gleich reines Material erhalten, sodass die Gesamtausbeute 90% betrug. IR, NMR und MS entsprachen.

## Beispiel 3

Carbocyclisches 2′,3′-Didehydro-2′,3′dideoxyguanosin

Eine Lösung von 13,32 g (±)-(1α,4α)-4-[(2-Amino-1,6-dihydro-5-nitro-6-oxo -4-pyrimidinyl)-amino]-2-cyclopentencarbinol, 335 ml DMF und 250 ml Methanol wurde zum Rückfluss erhitzt, während eine Lösung von 39,2 g $Na_2S_2O_4$ in 250 ml Wasser auf ein Mal zugegeben wurde. Die Suspension wurde nach und nach homogen, während sie 30 Minuten lang zum Rückfluss erhitzt wurde. TLC-Analyse (4:1 $CHCl_3$:MeOH) zeigte, dass das gesamte Ausgangsmaterial verbraucht worden war.

Das Gemisch, das (±)-(1α,4α)-[(2,5-Diamino-1,6-dihydro-6-oxo -4-pyrimidinyl)amino]-2-cyclopentencarbinol enthielt, wurde auf Raumtemperatur gekühlt und zu einem braungelben Rückstand konzentriert. Der Rückstand wurde in einem Gemisch von 250 ml wasserfreiem DMF und 250 ml Triäthylorthoformat suspendiert und mit 1,0 g p-Toluolsulfonsäure•$H_2O$ über Nacht unter Argon zum Rückfluss erhitzt. Das Gemisch wurde zu einem dunkelbraunen Feststoff konzentriert, der in 500 ml 0,5N HCl gelöst und 3 Stunden bei Raumtemperatur gerührt wurde.

Die wässrige Lösung wurde zu einem dunklen Feststoff konzentriert, der an 300 g Silicagel adsorbiert wurde, die mit $CHCl_3$:MeOH (4:1) eluiert wurden. Dabei wurden 15 g eines orangen Feststoffes erhalten. Der Feststoff wurde einer Flash-Chromatographie an Silicagel (30-40 μ) unterworfen und lieferte 5,2 g (40%) Carbovir vom Schmelzpunkt 245-246 °C. IR, NMR und MS entsprachen.

**Ansprüche**

1. Verfahren zur Herstellung von Carbovir, dadurch gekennzeichnet, dass man
a) eine Verbindung der Formel

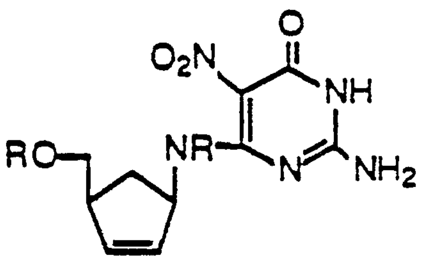

III

worin R Wasserstoff oder eine mit Säure spaltbare Schutzgruppe darstellt,
mit wässrigem Natriumdithionit in Gegenwart eines Alkanols und eines polaren aprotischen Lösungsmittels unter Erhitzen zum Rückfluss zu einer Verbindung der Formel

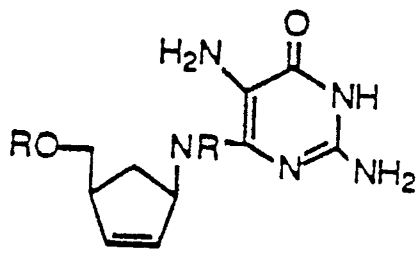

IV

worin R die obige Bedeutung hat,
reduziert und
b) die Verbindung der Formel IV durch
(i) Behandlung mit einem Formylierungsmittel in einem polaren aprotischen Lösungsmittel in Gegenwart eines nicht-wässrigen sauren Katalysators unter Erhitzen zum Rückfluss und
(ii) Dehydratisieren der formylierten Verbindung mit einer Mineralsäure
cyclisiert.

2. Verfahren nach Anspruch 1, wobei R Wasserstoff ist.

3. Verfahren nach Anspruch 1, wobei in Stufe (a) das Alkanol Methanol und das polare aprotische Lösungsmittel Dimethylformamid ist.

4. Verfahren nach Anspruch 1, wobei die Verbindung der Formel III dadurch hergestellt wird, dass man eine Verbindung der Formel

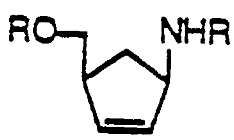

I

worin R die obige Bedeutung hat,
mit einer Verbindung der Formel

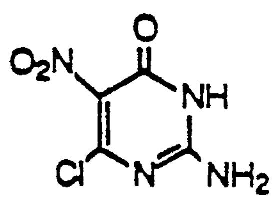

II

in einem polaren aprotischen Lösungsmittel in Gegenwart eines tertiären Alkylamins umsetzt.

5. Verfahren nach Anspruch 4, wobei R Wasserstoff ist.

6. Verfahren nach Anspruch 4, wobei das polare aprotische Lösungsmittel Dimethylformamid und das tert. Alkylamin Triäthylamin ist.

7. Verbindungen der Formel

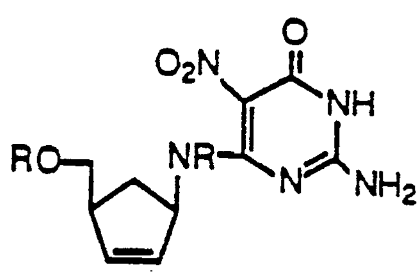

worin R Wasserstoff odere eine mit Säure spaltbare Schutzgruppe ist.

8. (±)-(1α,4α)-4-[(2-Amino-1,6-dihydro-5-nitro-6-oxo-4-pyrimidinyl)amino]-2-cyclopentencarbinol.

9. Verbindungen der Formel

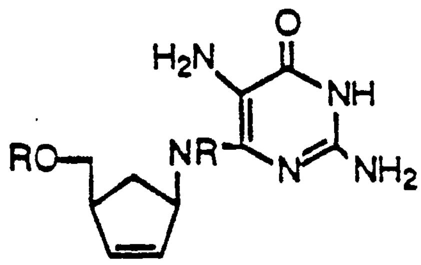

worin R Wasserstoff odere eine mit Säure spaltbare Schutzgruppe ist.

10. (±)-(1α,4α)-4-[(2,5-Diamino-1,6-dihydro-6-oxo-4-pyrimidinyl)amino]-2-cyclopentencarbinol.

Europäisches Patentamt

# EUROPÄISCHER RECHERCHENBERICHT

Nummer der Anmeldung

EP 90106989.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| P,A | EP - A1 - 0 346 132 (REGENTS OF THE UNIVERSITY OF MINNESOTA) * Beispiele; Ansprüche; Fig. 1 * -- | 1,4,7, 9 | C 07 D 473/18<br>C 07 D 239/50 |
| P,A | DE - A1 - 3 901 502 (REGENTS OF THE UNIVERSITY OF MINNESOTA) * Ansprüche; Beispiele; Seite 9, Zeilen 21-66 * -- | 1,4,7, 9 | |
| P,A | CHEMICAL ABSTRACTS, Band 112, Nr. 5, 29. Jänner 1990, Columbus, Ohio, USA R.VINCE et al. "Synthesis and anti-HIV activity of carbocyclic 2',3'-didehydro-2',3'-dideoxy 2,6-disubstituted purine nucleosides" Seite 632, Spalte 2, Zusammenfassung-Nr. 36 354p & J. Med. Chem. 1990, 33(1), 17-21 ---- | 1,4 | |
| | | | RECHERCHIERTE SACHGEBIETE (Int Cl 5) |
| | | | C 07 D 239/00<br>C 07 D 473/00 |

Der vorliegende Recherchenbericht wurde für alle Patentansprüche erstellt

| Recherchenort | Abschlußdatum der Recherche | Prüfer |
|---|---|---|
| VIENNA | 25-07-1990 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument